# EUROPEAN PATENT APPLICATION

(11) **EP 1 543 832 A2**
(43) Date of publication of application: **22.06.2005**
(21) Application number: 04019110.8
(22) Date of filing: 12.08.2004
(51) Int. Cl.: A61K 31/519, A61P 25/14

(54) **Use of trans-4[4-(3-chloro-4-methoxy-benzylamino) benzothieno[2,3-d]pyrimidin-2-yl]-cyclohexanecarboxylic acid in the treatment of extrapyramidal movement disorders**

(30) Priority: 16.12.2003 EP 03028860
(71) Applicant: MERCK PATENT GmbH, 64293 Darmstadt (DE)
(72) Inventor: Russ, Hermann, Dr., 60594 Frankfurt (DE); Bartoszyk, Gerd, Dr., 64331 Weiterstadt (DE)

(57) **Abstract**

Trans-4-[4-(3-chloro-4-methoxybenzylamino)-benzothieno-[2,3-d]pyrimidin-2-yl]cyclohexanecarboxylic acid or a physiologically acceptable salt thereof is used for the manufacture of a medicament for the treatment of extrapyramidal movement disorders and/or adverse effects in extrapyramidal movement disorders.

## Description

The present invention relates to the use of trans-4-[4-(3-chloro-4-methoxy-benzylamino)-benzothieno-[2,3-d]pyrimidin-2-yl]cyclohexanecarboxylic acid or a physiologically acceptable salt thereof for the manufacture of a medicament for the treatment of extrapyramidal movement disorders and/or for the manufacture of a medicament for the treatment of adverse effects of anti-Parkinsonian drugs in extrapyramidal movement disorders and/or for the manufacture of a medicament for the treatment of extrapyramidal symptoms (EPS) induced by neuroleptics.

Trans-4-[4-(3-chloro-4-methoxybenzylamino)-benzothieno-[2,3-d]pyrimidin-2-yl]cyclohexanecarboxylic acid and physiologically acceptable salts thereof as well as a process by which it can be prepared are known from WO 99/55708. Further processes for manufacturing the compound and precursors of it are described in WO 00/78767 and WO 01/42248.
The compounds which are referred to herein are described in the patent as PDE V inhibitors.
Therefore, the use of trans-4-[4-(3-chloro-4-methoxybenzylamino)-benzothieno-[2,3-d]pyrimidin-2-yl]cyclohexanecarboxylic acid and its physiologically acceptable acid addition salts for the manufacture of a medicament for treatment of diseases of the cardiovascular system, in particular cardiac insufficiency, and for the treatment and/or therapy of impaired potency (erectile dysfunction). Further uses are described in WO 01/19369.

The invention had the object of providing novel uses for trans-4-[4-(3-chloro-4-methoxybenzylamino)-benzothienio-[2,3-d]pyrimidin-2-yl]-cyclohexanecarboxylic acid and its physiologically acceptable salts.

It has been found that trans-4-[4-(3-chloro-4-methoxybenzylamino)-benzothieno-[2,3-d]pyrimidin-2-yl]cyclohexanecarboxylic acid or physiologically acceptable salts also has therapeutic activity against extrapyramidal movement disorders such as idiopathic Parkinsons's disease, Parkinson syndromes, dyskinetic, choreatic, or dystonic syndromes, tremor, Gilles de la Torette syndrome, ballism, myoclonus, restless legs syndrome or Wilsons's disease, as well as extrapyramidal motoric disturbances [synonymous extrapyramidal symptoms (EPS)] induced by neuroleptics.

Additionally it has been found that trans-4-[4-(3-chloro-4-methoxybenzyl-amino)-benzothieno-[2,3-d]pyrimidin-2-yl]cyclohexanecarboxylic acid or physiologically acceptable salts has therapeutic activity against adverse effects of anti-Parkinsonian drugs in extramyramidal movement disorders, in particular against dopaminomimetic adverse effects of anti-Parkinsonian drugs in idiopathic Parkinson's disease or Parkinson syndromes.

Furthermore it has been found that trans-4-[4-(3-chloro-4-methoxybenzyl-amino)-benzothieno-[2,3-d]pyrimidin-2-yl]cyclohexanecarboxylic acid or physiologically acceptable salts shows an extremely low liability to induce extrapyramidal side effects. Extrapyramidal motor side effects in e.g. rodents are measured by the ability of a drug to induce catalepsy. Catalepsy is defined as a state where an animal continues to remain in an unnormal (nonphysiological 'uncomfortable') posture for a long time (*e.g.*: M.E. Stanley and S.D. Glick, Neuropharmacology, 1996; 15: 393-394; C.J.E. Niemegeers and P. Janssen, Life Sci., 1979, 201-2216). For example, if a hindpaw of a rat is placed on an elevated level, *e.g.* a platform elevated 3 cm above ground level, a normal rat immediately withdraws the hindpaw from the platform to the ground level. A cataleptic rat remains in this unnatural posture even for minutes.

Unexpectedly, trans-4-[4-(3-chloro-4-methoxybenzylamino)-benzothieno-[2,3-d]pyrimidin-2-yl]cyclohexanecarboxylic acid or physiologically acceptable salts moreover are able to prevent catalepsy induced by conventional antidopaminergic drugs and even reverses already existing catalepsy induced by conventional antidopaminergic drugs such as haloperidol.

The present invention relates to the use of trans-4-[4-(3-chloro-4-methoxybenzylamino)-benzothieno-[2,3-d]pyrimidin-2-yl]cyclohexane-carboxylic acid or physiologically acceptable salts thereof for the manufacture of a medicament for the treatment of extrapyramidal movement disorders.

A preferred salt of trans-4-[4-(3-chloro-4-methoxybenzylamino)-benzothieno-[2,3-d]pyrimidin-2-yl]cyclohexanecarboxylic acid is the sodium or ethanolamine salt.

Therefore, the invention relates to the use for the manufacture of a medicament for the treatment of extrapyramidal movement disorders in which the pharmacologically acceptable salt is trans-4-[4-(3-chloro-4-methoxybenzylamino)-benzothieno-[2,3-d]pyrimidin-2-yl]cyclohexane-carboxylic acid sodium salt or ethanolamine salt.

Additionally, the invention relates to the use of a pharmaceutical composition containing at least one compound of trans-4-[4-(3-chloro-4-methoxybenzylamino)-benzothieno-[2,3-d]pyrimidin-2-yl]cyclohexane-carboxylic acid or one of their biocompatible salts together with at least one solid, liquid or semiliquid excipient or adjunct for the treatment of extrapyramidal movement disorders.

Trans-4-[4-(3-chloro-4-methoxybenzylamino)-benzothieno-[2,3-d]pyrimidin-2-yl]cyclohexanecarboxylic acid or their physiologically acceptable salts, useful for the treatment of extrapyramidal movement disorders, in particular for the treatment of idiopathic Parkinson's disease, Parkinson syndromes, dyskinetic, choreatic or dystonic syndromes, extrapyramidal motoric adverse effects of neuroleptics, tremor, Gilles de la Tourette syndrome, ballism, myoclonus, restless legs syndrome or Wilson's disease and/or useful for the treatment of adverse effects in idiopathic Parkinson's disease or Parkinson syndromes including medicinal compositions as defined below, is preferably administered in doses from 0.1 to 100 mg, preferentially between approximately 1 and 20 mg. The composition may be administered once or more times a day, e.g. 2, 3, or 4 times daily.

Anti-Parkinsonian drugs are conventional drugs such as I-dopa (levodopa) and I-dopa combined with benserazide or carbidopa, dopamine agonists such as bromocriptine, apomorphine, cabergoline, pramipexol, ropinirol, pergolide, dihydro-α-ergocriptine or lisuride plus all drugs acting via stimulation of dopamine receptors, inhibitors of catechol-O-methyl transferase (COMT) such as entacapone or tolcapone, inhibitors of monoamine oxidase (MAO) such as selegiline and antagonists of N-methyl-D-aspartate (NMDA) receptors such as amantadine or budipine.

Adverse effects of said anti-Parkinsonian drugs are all types of dyskinesias, such as choreic, dystonic, ballistic and myoclonic dyskinesia, as well as motor (response) fluctuations or psychotic states.

Therefore, the present invention relates to the use of trans-4-[4-(3-chloro-4-methoxybenzylamino)-benzothieno-[2,3-d]pyrimidin-2-yl]cyclohexanecarboxylic acid or a physiologically acceptable salt thereof for the manufacture of a medicament for the treatment of adverse effects of anti-Parkinsonian drugs in idiopathic Parkinson's disease.

Treatment of adverse effects of conventional anti-Parkinsonian drugs as defined above are determined in a modification of the animal model of the Parkinsonian cynomolgus monkey according to P.J. Blanchet et al., Exp. Neurology 1998; 153: 214-222. Monkeys render parkinsonian by repeated injections of 1-methyl-4-phenyl-1,2,3,6-tetrahydropyridine (MPTP). The Parkinsonian monkeys are chronically treated with the standard I-dopa therapy according to P.J. Blanchet *et al.,* Mov. Disord., 1998; **13:** 798-802. Longterm treatment with I-dopa induces extrapyramidal motor side effects and psychotic states which are both qualitatively and quantitatively, assessed by the Abnormal Involuntary Movement Scale (P.J. Blanchet *et al.,* Mov. Disord. 1998; **13:** 798-802) for different body parts (face, neck, trunk, each limb) and by rating for psychotic states by observing the monkey's attention, reactivity and mobility. Trans-4-[4-(3-chloro-4-methoxybenzylamino)-benzothieno-[2,3-d]pyrimidin-2-yl]cyclohexane-carboxylic acid reduced overall choreiform dyskinesias and dystonic dyskinesias as well as psychotic states.

A typical study to investigate the efficacy of the compounds according to the invention for adverse effects in Parkinson's disease is described in the following. 40 patients of either sex with advanced idiopathic Parkinson's disease complicated by "peak-dose" dyskinesia participate in a double-blind, cross-over study. The main inclusion criteria are Hoehn & Yahr stage ≥ 2.5 (lit.: Hoehn H.M. et al, Neurology 1967; 17: 427-442), aged 40-75 years, symptom duration of at least 5 years, and a I-dopa treatment duration of at least 3 years. trans-4-[4-(3-chloro-4-methoxybenzylamino)-benzothieno-[2,3-d]pyrimidin-2-yl]cyclohexanecarboxylic acid sodium or ethanolamine salt or placebo is administered as "add on" to the conventional Parkinson treatment; which is maintained unchanged during the whole study. The dose of blinded medication is titrated over a period of 3 weeks in a range from 2.5 to 10 mg b.i.d. Then the medication is kept constant for 1 week. Before the start of titration and at the end of the treatment period a I-dopa challenge is performed according to P. Damier et al. (Movement Disord,1999, **14** (Suppl. 1), 54-59) using video recording. The main outcome measure of the protocol is the mean score for dyskinesia during the first hour in the "on" state after I-dopa challenge. Therefore, the investigator assesses every minute the severity of dyskinesia (0 = absent, 4 = severe disabling involuntary movements) from 0 to 4 in seven parts of the body (upper and lower limbs, face, trunk, neck). After a 2-week wash-out period the two study arms are crossed over and the protocol is repeated. The statistical analysis of the mean dyskinesia scores demonstrates a significant clinical improvement under treatment with trans-4-[4-(3-chloro-4-methoxybenzylamino)-benzothieno-[2,3-d]pyrimidin-2-yl]cyclohexanecarboxylic acid sodium or ethanolamine salt.

Additionally, the invention relates to the use of a pharmaceutical composition containing at least one compound of trans-4-[4-(3-chloro-4-methoxybenzylamino)-benzothieno-[2,3-d]pyrimidin-2-yl]cyclohexane-carboxylic acid or one of its biocompatible salts together with at least one solid, liquid or semiliquid excipient or adjunct for the treatment of adverse effects of anti-Parkinsonian drugs in idiopathic Parkinson's disease.

Furthermore, the present invention relates to the use of trans-4-[4-(3-chloro-4-methoxybenzylamino)-benzothieno-[2,3-d]pyrimidin-2-yl]cyclohexanecarboxylic acid or one of its biocompatible salts for the manufacture of a medicament for the treatment of idiopathic Parkinson's disease.

A typical animal model for idiopathic Parkinson's disease is the Parkinsonian cynomolgus monkey according to P.J. Blanchet *et al.,* Exp. Neurology 1998; **153:** 214-222. Monkeys render parkinsonian by repeated injections of 1-methyl-4-phenyl-1,2,3,6-tetrahydropyridine (MPTP).

Parkinsonian symptoms are qualitatively assessed by the use of the Laval University Disability Scale (B. Gomez-Mancilla *et al.,* 1993; Mov. Disord. **8**: 144-150) measuring the following symptoms: posture, mobility, climbing, gait, holding food, vocalizing, grooming, social interaction. Trans-4-[4-(3-chloro-4-methoxybenzylamino)-benzothieno-[2,3-d]pyrimidin-2-yl]cyclo-hexanecarboxylic acid reduced all the parkinsonian symptoms and increased total activity.

A typical study to investigate the efficacy of the compounds according to the invention in the treatment of idiopathic Parkinson's disease is described in the following. 180 patients of either sex with idiopathic Parkinson's disease participate in a double-blind study. The main inclusion criteria are Hoehn & Yahr stage ≥ 2.0 (Hoehn H.M. et al, Neurology 1967; 17: 427-442), aged 50-80 years, symptom duration of at least 5 years. Trans-4-[4-(3-chloro-4-methoxybenzylamino)-benzothieno-[2,3-d]pyrimidin-2-yl]cyclohexanecarboxylic acid or placebo is administered as "add on" to the conventional Parkinson treatment, which is maintained unchanged during the whole study. The dose of blinded medication is titrated over a period of 4 weeks in a range from 2.5 to 10 mg b.i.d. Then the medication is kept constant for 1 week. Before the start of titration, at the end of the treatment period and 2 weeks after the end of the tiration period an assessment is performed in each patients using the unified Parkinson's disease rating scale (UPDRS part I to V according to S. Fahn et al., in: Recent developments in Parkinson's disease, vol. **2**, MacMillan health information 1987, 153-163). This allows to detect simultaneously a beneficial effect of trans-4-[4-(3-chloro-4-methoxybenzylamino)-benzothieno-[2,3-d]pyrimidin-2-yl]cyclohexanecarboxylic acid or one of its biocompatible salts, in particular of trans-4-[4-(3-chloro-4-methoxybenzyl-amino)-benzothieno-[2,3-d]pyrimidin-2-yl]cyclohexanecarboxylic acid sodium or ethanolamine salt, on the global motoric function, on dystonia, motor fluctuations, and on psychosis. Furthermore, the efficacy to treat tremor is shown by the means of the UPDRS. The statistical analysis of the UPDRS scores demonstrates a significant clinical improvement under treatment with trans-4-[4-(3-chloro-4-methoxybenzylamino)-benzothieno-[2,3-d]pyrimidin-2-yl]cyclohexanecarboxylic acid sodium or ethanolamine salt.

Therefore the invention relates to the use for the manufacture of a medicament for the treatment of idiopathic Parkinson's disease in which the physiologically acceptable salt is trans-4-[4-(3-chloro-4-methoxybenzyl-amino)-benzothieno-[2,3-d]pyrimidin-2-yl]cyclohexanecarboxylic acid sodium or ethanolamine salt.

Additionally, the invention relates to the use of a pharmaceutical composition containing at least one compound of trans-4-[4-(3-chloro-4-methoxybenzylamino)-benzothieno-[2,3-d]pyrimidin-2-yl]cyclohexane-carboxylic acid or one of its biocompatible salts together with at least one solid, liquid or semiliquid excipient or adjunct for the treatment of idiopathic Parkinson's disease.

The limiting factor of Parkinson treatment with I-dopa and/or dopamine agonists is often the occurence of psychosis or dyskinesia and other motor fluctuations.

It has been found that trans-4-[4-(3-chloro-4-methoxybenzylamino)-benzothieno-[2,3-d]pyrimidin-2-yl]cyclohexanecarboxylic acid or a physiologically acceptable salt thereof enhance the anti-Parkinsonian effect of anti-Parkinsonian drugs as defined above without inducing extrapyramidal side effects.

Therefore, the add-on therapy with trans-4-[4-(3-chloro-4-methoxybenzyl-amino)-benzothieno-[2,3-d]pyrimidin-2-yl]cyclohexanecarboxylic acid or a physiologically acceptable salt thereof, in particular of their hydrochlorides, now opens the possibility to increase the doses of I-dopa and/or dopamine agonists and/or all other anti-Parkinsonian drugs as defined above in order to counteract periods of insufficient motility ("off" phases) without provoking the above mentioned side effects. That represents an entirely novel approach in the treatment of Parkinson's disease leading to a significant benefit for the patients.

Thus, the invention relates to a pharmaceutical composition comprising, as active principles, (i) trans-4-[4-(3-chloro-4-methoxybenzylamino)-benzothieno-[2,3-d]pyrimidin-2-yl]cyclohexanecarboxylic acid or a physiologically acceptable salt thereof, and (ii) at least one anti-Parkinsonian drug, in combination with one or more pharmaceutically acceptable excipients.

Particularly, the invention relates to a pharmaceutical composition comprising, as active principles, (i) trans-4-[4-(3-chloro-4-methoxybenzylamino)-benzothieno-[2,3-d]pyrimidin-2-yl]cyclohexane-carboxylic acid sodium or ethanolamine salt, and (ii) I-dopa or I-dopa combined with benserazide or carbidopa, in combination with one or more pharmaceutically acceptable excipients.

The ratios of the respective amounts of trans-4-[4-(3-chloro-4-methoxy-benzylamino)-benzothieno-[2,3-d]pyrimidin-2-yl]cyclohexanecarboxylic acid or on of its physiologically acceptable salts and of the conventional anti-Parkinsonian drug thus vary in consequences.
Preferably, the weight ratio of trans-4-[4-(3-chloro-4-methoxybenzylamino)-benzothieno-[2,3-d]pyrimidin-2-yl]cyclohexanecarboxylic acid or one of its physiologically acceptable salts to the conventional anti-Parkinsonian drug ranges from 1:1 to 1:100, preferably from 1:10 to 1:90 and better still from 1:40 to 1:60.

Another subject of the present invention is additionally the use of trans-4-[4-(3-chloro-4-methoxybenzylamino)-benzothieno-[2,3-d]pyrimidin-2-yl]-cyclohexanecarboxylic acid or one of its physiologically acceptable salts in combination with at least one anti-Parkinsonian drug, for the preparation of a medicinal combination intended to enhance the anti-Parkinsonian effect of said anti-Parkinsonian drugs.

According to the invention, the term "medicinal combination" is inteded to refer either to a pharmaceutical composition as defined above, in which the two active principles or compounds are the essential constituents of the same composition, or to a kit comprising two separate compositions, the first comprising trans-4-[4-(3-chloro-4-methoxybenzylamino)-benzothieno-[2,3-d]pyrimidin-2-yl]cyclohexanecarboxylic acid or one of its physiologically acceptable salts as sole active principle, and the second comprising at least one anti-Parkinsonian drug as active compound.

When the medicinal combination is in the form of a kit, the administration of the two compositions constituting this kit, although carried out separately, is simultaneous for a combined therapy. It is preferred to use in the form of the sodium or ethanolamine salt.

Adverse effects of anti-Parkinsonian drugs as defined above are additionally known in particular in Parkinson syndromes.

Parkinson syndromes are e.g. multiple system atrophies (MSA), Steele-Richardson-Olszewski syndrome (= progressive supranuclear palsy), cortico-basal degeneration, olivo-ponto cerebellar atrophy or Shy Drager syndrome.
Trans-4-[4-(3-chloro-4-methoxybenzylamino)-benzothieno-[2,3-d]pyrimidin-2-yl]cyclohexanecarboxylic acid or a physiologically acceptable salt thereof are useful for the treatment of Parkinson syndromes in particular of multiple system atrophies.

Therefore the present invention relates to the use of trans-4-[4-(3-chloro-4-methoxybenzylamino)-benzothieno-[2,3-d]pyrimidin-2-yl]cyclohexane-carboxylic acid or a physiologically acceptable salt thereof, for the manufacture of a medicament for the treatment of adverse effects in Parkinson syndromes.

The present invention relates additionally to the use of trans-4-[4-(3-chloro-4-methoxybenzylamino)-benzothieno-[2,3-d]pyrimidin-2-yl]cyclohexane-carboxylic acid or a physiologically acceptable salt thereof, for the manufacture of a medicament for the treatment of Parkinson syndromes.

A typical animal model is the reserpinized rat or mouse (*e.g.* M.S. Starr and B.S. Starr, J. Neural Transm. - Park. Dis. Dement. Sect., 1994; **7**: 133-142; M. Gossel et al., J. Neural Transm. - Park. Dis. Dement. Sect., 1995; **10**: 27-39; N.R. Hughes *et al.,* Mov. Disord., 1998; 13: 228-233). Reserpine is a potent depleter of monoamines and produces nearly complete akinesia in both species. Prominent 24 h after application, the distance travelled and the time active is nearly zero as measured in conventional activity meters. Trans-4-[4-(3-chloro-4-methoxybenzylamino)-benzothieno-[2,3-d]pyrimidin-2-yl]cyclohexanecarboxylic acid or a physiologically acceptable salt thereof dose-dependently reduced akinesia, *i.e.* restored distance travelled and time active to about the level of normal animals.

Another more recent animal model is the striatonigral degeneration approach in the rat according to G.K. Wenning *et al.,* J. Neural Transm. Suppl., 1999; **55:** 103-113. Rats receive an unilateral injection of 6-hydroxydopamine into the left medial forebrain bundle followed by an injection of quinolinic acid into the ipsilateral striatum inducing nigrostriatal degeneration. The degeneration results in turning behavior to a challenge with dopaminomimetics such as apomorphine or amphetamine. Turning behavior is measured by an automated recorder. Turning behavior induced by apomorphine or amphetamine is dose-dependently antagonized by trans-4-[4-(3-chloro-4-methoxybenzylamino)-benzothieno-[2,3-d]pyrimidin-2-yl]cyclohexanecarboxylic acid or a physiologically acceptable salt thereof.

Multiple system atrophy (MSA) is due to an expansive neurodegeneration in the extrapyramidal and autonomic nervous system which leads to an akinetic Parkinsonian syndrome with vegetative disturbances. In contrast to idiopathic Parkinson's disease the density of central dopamine receptors is markedly decreased and therefore, MSA patients poorly respond to dopaminergic drugs. Since trans-4-[4-(3-chloro-4-methoxybenzylamino)-benzothieno-[2,3-d]pyrimidin-2-yl]cyclohexanecarboxylic acid or a physiologically acceptable salt thereof act predominantly via serotonin receptors on the extrapyramidal system, they are able to improve the motor performance in these otherwise mostly untreatable patients.

A typical study to investigate the efficacy of the compounds according to the invention in MSA patients encompasses 30 patients of either sex with a symptom duration of at least 5 years and a significant reduction of central dopamine receptors in positron emission tomography (PET) scan. The study design is similar to that described above for Parkinson's disease. Trans-4-[4-(3-chloro-4-methoxybenzylamino)-benzothieno-[2,3-d]pyrimidin-2-yl]cyclohexanecarboxylic acid sodium or ethanolamine salt or placebo is titrated as "add on" to the conventional treatment (dose range 2.5 to 20 mg b.i.d.). Before the start of titration and at the end of the treatment period a complete UPDRS assessment is performed in each patient (primary outcome measure). After a 2-week wash-out period the two study arms are crossed over and the protocol is repeated. Statistical analysis of UPDRS demonstrates a significant clinical improvement under treatment with trans-4-[4-(3-chloro-4-methoxybenzylamino)-benzothieno-[2,3-d]pyrimidin-2-yl]cyclohexanecarboxylic acid sodium or ethanolamine salt.

Therefore, the invention relates to the use for the manufacture of a medicament for the treatment of adverse effects of anti-Parkinsonian drugs in Parkinson syndromes in which the pharmacologically acceptable salt is trans-4-[4-(3-chloro-4-methoxybenzylamino)-benzothieno-[2,3-d]pyrimidin-2-yl]cyclohexanecarboxylic acid sodium or ethanolamine salt.

Additionally, the invention relates to the use of a pharmaceutical composition containing at least one compound of trans-4-[4-(3-chloro-4-methoxybenzylamino)-benzothieno-[2,3-d]pyrimidin-2-yl]cyclohexane-carboxylic acid or a physiologically acceptable salt thereof together with at least one solid, liquid or semiliquid excipient or adjunct for the treatment of adverse effects of anti-Parkinsonian drugs in Parkinson syndromes.

Additionally, the invention relates to the use of a pharmaceutical composition containing at least one compound of trans-4-[4-(3-chloro-4-methoxybenzylamino)-benzothieno-[2,3-d]pyrimidin-2-yl]cyclohexane-carboxylic acid or a physiologically acceptable salt thereof together with at least one solid, liquid or semiliquid excipient or adjunct for the treatment of Parkinson syndromes.

The present invention relates to the use of trans-4-[4-(3-chloro-4-methoxybenzylamino)-benzothieno-[2,3-d]pyrimidin-2-yl]cyclohexane-carboxylic acid or a physiologically acceptable salt thereof, for the manufacture of a medicament for the treatment of dyskinetic and/or choreatic syndromes.

Dyskinetic and/or choreatic syndromes are e.g. Huntington's disease, minor chorea or chorea of pregnancy. trans-4-[4-(3-chloro-4-methoxybenzylamino)-benzothieno-[2,3-d]pyrimidin-2-yl]cyclohexanecarboxylic acid or a physiologically acceptable salt thereof are in particular useful for the treatment of Huntington's disease.

A typical animal model is the systemic 3-nitropropionic acid (3-NP) model in rats according to C.V. Borlongan *et al.,* Brain Res., 1995; **697:** 254-257. Rats are treated with injections of the selective striatal neurotoxin 3-NP i.p. every fourth day (C.V. Borlongan *et al.,* Brain Res. Protocols, 1997; 1: 253-257). After two injections of 3-NP, rats display nocturnal hyperactivity reflecting symptoms of early Huntington's disease, whereas rats treated with four injections of 3-NP display nocturnal akinesia (hypoactivity) reflecting symptoms of late Huntington's disease. Nocturnal activity is automatically measured in conventional acitivity cages by infrared beams. Trans-4-[4-(3-chloro-4-methoxybenzylamino)-benzothieno-[2,3-d]pyrimidin-2-yl]cyclohexanecarboxylic acid or a physiologically acceptable salt thereof reduce both the nocturnal hyperactivity and akinesia.

A typical trial to establish the effect of the compounds according to the invention on chorea, voluntary motor performance, and functional disability in patients with Huntington's disease encompasses 32 genetically diagnosed patients. Trans-4-[4-(3-chloro-4-methoxybenzylamino)-benzothieno-[2,3-d]pyrimidin-2-yl]cyclohexanecarboxylic acid sodium or ethanolamine salt or placebo is administered as "add on" to the conventional treatment, which is maintained unchanged during the whole study. The dose of blinded medication is titrated over a period of 3 weeks in a range from 2.5 to 20 mg b.i.d. Then the medication is held constant for 1 week. Assessments are performed in the week before and at the last day of the trial. Chorea is scored using the abnormal involuntary movement scale (AIMS, W. Guy, in: ECDEU assessment manual. Rockville MD: US dept. of health, education and welfare, 1976: 534-537), the unified Huntington's disease rating scale (UHDRS, Huntington study group, 1996, Movement Disord, 11: 136-42), and judgement of video recordings. Voluntary motor performance is assessed using the UHDRS motor scale. Patients and their partners complete a questionnaire regarding functional disability. Statistical analysis demonstrates significant improvement of voluntary and involuntary motor performance in Huntington patients under treatment with trans-4-[4-(3-chloro-4-methoxybenzylamino)-benzothieno-[2,3-d]pyrimidin-2-yl]cyclohexanecarboxylic acid or a physiologically acceptable salt thereof.

A preferred salt of trans-4-[4-(3-chloro-4-methoxybenzylamino)-benzothieno-[2,3-d]pyrimidin-2-yl]cyclohexanecarboxylic acid is the sodium or ethanolamine salt.

Therefore, the invention relates to the use for the manufacture of a medicament for the treatment of dyskinetic and/or choreatic syndromes, in particular for the treatment of Huntington's disease, in which the pharmacologically acceptable salt is trans-4-[4-(3-chloro-4-methoxybenzylamino)-benzothieno-[2,3-d]pyrimidin-2-yl]cyclohexane-carboxylic acid sodium or ethanolamine salt.

Additionally, the invention relates to the use of a pharmaceutical composition containing at least one compound of or a physiologically acceptable salt thereof together with at least one solid, liquid or semiliquid excipient or adjunct for the treatment of dyskinetic and/or choreatic syndromes.

The present invention relates to the use of or a physiologically acceptable salt thereof, for the manufacture of a medicament for the treatment of dystonic syndromes.

Dystonic syndromes are e.g. spasmalic torticollis, writer's cramp, blepharospasm, Meige syndrome or dopasensitive dystonia. Trans-4-[4-(3-chloro-4-methoxybenzylamino)-benzothieno-[2,3-d]pyrimidin-2-yl]cyclohexanecarboxylic acid or a physiologically acceptable salt thereof is in particular useful for the treatment of spasmalic torticollis and/or blepharospasm.

A typical animal model is the mutant dystonic hamster according to A. Richter and W. Löscher, Prog. Neurobiol. 1998; **54:** 633-677. In this genetically dystonic hamsters, dystonic attacks are provoked by taking the animal from the home cage and placing it on a balance. The dystonic syndrome consists of a sequence of abnormal movements, and the severity of the single symptoms is rated by a scoring system. Trans-4-[4-(3-chloro-4-methoxybenzylamino)-benzothieno-[2,3-d]pyrimidin-2-yl]cyclo-hexanecarboxylic acid or a physiologically acceptable salt thereof dose-dependently reduce the severity of dystonic symptoms.

To demonstrate the efficacy of the compounds according to the invention in dystonic syndromes, a double-blind, placebo-controlled study is performed in patients with cervical dystonia (spasmodic torticollis) who do not tolerate injection of botulinum toxin. Trans-4-[4-(3-chloro-4-methoxybenzylamino)-benzothieno-[2,3-d]pyrimidin-2-yl]cyclohexanecarboxylic acid sodium or ethanolamine salt is titrated as described above in the range from 2.5 mg to 20 mg b.i.d. The Toronto western spasmodic torticollis rating scale (TWSTRS, C.L. Comella et al., 1997, Movement Disord, **12**: 570-575) is used as primary outcome measure. A significant improvement in the TWSTRS scores is noted for the patients treated with trans-4-[4-(3-chloro-4-methoxybenzylamino)-benzothieno-[2,3-d]pyrimidin-2-yl]cyclohexane-carboxylic acid or a physiologically acceptable salt thereof.

A preferred salt of trans-4-[4-(3-chloro-4-methoxybenzylamino)-benzothieno-[2,3-d]pyrimidin-2-yl]cyclohexanecarboxylic acid is sodium or ethanolamine salt.

Therefore the invention relates to the use for the manufacture of a medicament for the treatment of dystonic syndromes, in particular of spasmalic torticollis and/or blepharospasm, in which the pharmacologically acceptable salt is trans-4-[4-(3-chloro-4-methoxybenzylamino)-benzothieno-[2,3-d]pyrimidin-2-yl]cyclohexanecarboxylic acid sodium or ethanolamine salt.

Additionally, the invention relates to the use of a pharmaceutical composition containing at least one compound of trans-4-[4-(3-chloro-4-methoxybenzylamino)-benzothieno-[2,3-d]pyrimidin-2-yl]cyclohexane-carboxylic acid or a physiologically acceptable salt thereof together with at least one solid, liquid or semiliquid excipient or adjunct for the treatment of dystonic syndromes.

The present invention relates to the use of trans-4-[4-(3-chloro-4-methoxybenzylamino)-benzothieno-[2,3-d]pyrimidin-2-yl]cyclohexane-carboxylic acid or a physiologically acceptable salt thereof, for the manufacture of a medicament for the treatment of extrapyramidal symptoms induced by neuroleptics.

Extrapyramidal motoric disturbances induced by neuroleptics are e.g. early dyskinesia, dystonia, akathisia, parkinsonoid, in particular bradykinesia, or tardive dyskinesia. Trans-4-[4-(3-chloro-4-methoxybenzylamino)-benzothieno-[2,3-d]pyrimidin-2-yl]cyclohexanecarboxylic acid or a physiologically acceptable salt thereof are useful particularly for the treatment of akathisia and/or tardive dyskinesia and/or parkinsonoid.

A typical animal model is neuroleptics-induced muscle rigidity in rats according to S. Wolfarth *et al.,* Arch. Pharmacol. 1992; 345: 209-212. Rats are challenged with the conventional neuroleptic drug haloperidol which enhances muscle tone. Muscle tone is electromechanically measured as the resistence to passive flexion and extension of the hind limb. Trans-4-[4-(3-chloro-4-methoxybenzylamino)-benzothieno-[2,3-d]pyrimidin-2-yl]cyclo-hexanecarboxylic acid or a physiologically acceptable salt thereof thereof decrease the mucle tone enhanced by haloperidol.

Another typical animal model is the neuroleptics sensitized monkey according to D.E. Casey, Psychopharmacology, 1996; **124**: 134-140. Monkeys treated repeatedly with conventional neuroleptics are highly sensitive to a subsequent challenge dose of neuroleptic drugs. When challenged, the monkeys immediately show extrapyramidal motor side effects such as dystonia, dyskinesias, akathisia, and bradykinesia which are rated by a scoring system. The conventional neuroleptic drug haloperidol is given as a challenge. When the before-mentioned extrapyramidal motor side effects occur, trans-4-[4-(3-chloro-4-methoxybenzyl-amino)-benzothieno-[2,3-d]pyrimidin-2-yl]cyclohexanecarboxylic acid or a physiologically acceptable salt thereof is administered; trans-4-[4-(3-chloro-4-methoxybenzylamino)-benzothieno-[2,3-d]pyrimidin-2-yl]cyclohexane-carboxylic acid dose-dependently reduce the extrapyramidal motor side effects.

Tardive dyskinesia is a common adverse effect of long-term treatment with neuroleptics. A typical study to investigate the efficacy of the compounds according to the invention in tardive dyskinesia is described in the following. 32 schizophrenic (DSM-III-R) inpatients aged 25 - 60 years on long-term stable antipsychotic treatment (duration of at least 5 years) entered the study. Trans-4-[4-(3-chloro-4-methoxybenzylamino)-benzothieno-[2,3-d]pyrimidin-2-yl]cyclohexanecarboxylic acid sodium or ethanolamine salt or placebo is administered as "add on" to the antipsychotic treatment, which is kept constant during the whole study. The dose of blinded medication is titrated over a period of 3 weeks in a range from 2.5 to 20 mg b.i.d. Then the medication is maintained under double-blind conditions for 2 weeks. After a 2-week wash-out period, the test drugs are crossed over. Assessments of tardive dyskinesia by means of the Abnormal Involuntary Movement Scale (AIMS, see obove) and of Parkinsonian extrapyramidal side effects (UPDRS, see above) are made pretreatment and posttreatment. AIMS scores during treatment with trans-4-[4-(3-chloro-4-methoxybenzylamino)-benzothieno-(2,3-d]pyrimidin-2-yl]cyclohexane-carboxylic acid sodium or ethanolamine salt are significantly lower than during placebo period.

A preferred salt of trans-4-[4-(3-chloro-4-methoxybenzylamino)-benzo-thieno-[2,3-d]pyrimidin-2-yl]cyclohexanecarboxylic acid is sodium or ethanolamine salt.

Therefore the invention relates to the use for the manufacture of a medicament for the treatment of extrapyramidal symptoms induced by neuroleptics, in particular of akathisia and/or tardive dyskinesia, in which the pharmacologically acceptable salt is trans-4-[4-(3-chloro-4-methoxybenzylamino)-benzothieno-[2,3-d]pyrimidin-2-yl]cyclohexane-carboxylic acid sodium or ethanolamine salt.

Additionally, the invention relates to the use of a pharmaceutical composition containing at least one compound of or a physiologically acceptable salt thereof together with at least one solid, liquid or semiliquid excipient or adjunct for the treatment of extrapyramidal symptoms induced by neuroleptics.

The present invention relates to the use of trans-4-[4-(3-chloro-4-methoxybenzylamino)-benzothieno-[2,3-d]pyrimidin-2-yl]cyclohexane-carboxylic acid or a physiologically acceptable salt thereof, for the manufacture of a medicament for the treatment of tremor.

Tremor includes all types of tremors such as essential tremor, activated physiological tremor, cerebellar tremor, orthostatic tremor or drug-induced tremor. trans-4-[4-(3-chloro-4-methoxybenzylamino)-benzothieno-[2,3-d]pyrimidin-2-yl]cyclohexanecarboxylic acid or a physiologically acceptable salt thereof are particularly useful for the treatment of essential tremor and/or drug-induced tremor.

Typical animal models utilize either genetic mutant animals or are models where tremor is induced by a pharmacological agent (for review: H. Wilms *et al.,* Mov. Disord., 1999; **14:** 557-571).

Typical genetic models in mutant animals are the Campus Syndrome in the Pietrain pig according to A. Richter *et al.* (Exp. Neurology, 1995; **134:** 205-213) or the Weaver mutant mouse according to J.R. Simon and B. Ghetti (Mol. Neurobiol., 1994; 9: 183-189). In the Campus Syndrome model, these mutant pigs show a high-frequency tremor when standing and during locomotion, but not while lying at rest. Assessment of tremor is made by accelerometric recording. In the Weaver mutant mouse, degenerative cerebellar atrophy is fould in association with tremor, gait instability, and toppling over the sides after a few steps. Gait disability and toppling result in dramatically reduced locomotor activity measured by the distance travelled and the time spent with ambulation in conventional activity cages.

Trans-4-[4-(3-chloro-4-methoxybenzylamino)-benzothieno-[2,3-d]pyrimidin-2-yl]cyclohexanecarboxylic acid or a physiologically acceptable salt thereof improve the Campus Syndrome in the Pietrain pig, *i.e.* reduce disabling tremor when standing and during locomotion, and enhance locomotor activity in the Weaver mutant mouse.

A typical animal model for drug-induced tremors is the oxotremorine-induced tremor (*e.g.* H. Hallberg and O. Almgren, Acta Physiol. Scand., 1987; **129**: 407-13; J.G. Clement and W.R. Dyck, J. Pharmacol. Meth., 1989; **22**: 25-36). Oxotremorine induces tremor which is measured by a rating scale. Trans-4-[4-(3-chloro-4-methoxybenzylamino)-benzothieno-[2,3-d]pyrimidin-2-yl]cyclohexanecarboxylic acid or a physiologically acceptable salt thereof inhibit oxotremorine-induced tremors.

A preferred salt of trans-4-[4-(3-chloro-4-methoxybenzylamino)-benzothieno-[2,3-d]pyrimidin-2-yl]cyclohexanecarboxylic acid is sodium or ethanolamine salt.

Therefore the invention relates to the use for the manufacture of a medicament for the treatment of tremors, in particular of essential tremors and/or drug-induced tremors, in which the pharmacologically acceptable salt is trans-4-[4-(3-chloro-4-methoxybenzylamino)-benzothieno-(2,3-d]pyrimidin-2-yl]cyclohexanecarboxylic acid sodium or ethanolamine salt.

Additionally, the invention relates to the use of a pharmaceutical composition containing at least one compound of trans-4-[4-(3-chloro-4-methoxybenzylamino)-benzothieno-[2,3-d]pyrimidin-2-yl]cyclohexane-carboxylic acid or a physiologically acceptable salt thereof together with at least one solid, liquid or semiliquid excipient or adjunct for the treatment of tremor.

The present invention relates to the use of trans-4-[4-(3-chloro-4-methoxybenzylamino)-benzothieno-[2,3-d]pyrimidin-2-yl]cyclohexane-carboxylic acid or a physiologically acceptable salt thereof, for the manufacture of a medicament for the treatment of extrapyramidal movement disorders chosen from the group consisting of Gilles de la Tourette syndrome, ballism, myoclonus, restless legs syndrome and Wilson's disease.

A typical animal model for myoclonus is myoclonus induced by an acute hypoxic episode according to D.D. Truong *et al.,* Mov. Dsiord., 1994; **9**: 201-206). In this model of posthypoxic myoclonus, rats undergo a cardiac arrest for 8 minutes and are resuscitated thereafter. Myoclonic jerks occur spontaneously but can be provoked by auditory stimulation, too, worsening over the days following cardiac arrest. Trans-4-[4-(3-chloro-4-methoxy-benzylamino)-benzothieno-[2,3-d]pyrimidin-2-yl]cyclohexanecarboxylic acid or a physiologically acceptable salt thereof dose-dependently reduce the number of spontaneous and autitory-evoked myoclonic jerks.

A preferred salt of trans-4-[4-(3-chloro-4-methoxybenzylamino)-benzothieno-[2,3-d]pyrimidin-2-yl]cyclohexanecarboxylic acid is sodium or ethanolamine salt.

Therefore the invention relates to the use for the manufacture of a medicament for the treatment of extrapyramidal movement disorders chosen from the group consisting of Gilles de la Tourette syndrome, ballism, myoclonus, restless legs syndrome and Wilson's disease in which the pharmacologically acceptable salt is trans-4-[4-(3-chloro-4-methoxybenzylamino)-benzothieno-[2,3-d]pyrimidin-2-yl]cyclohexane-carboxylic acid sodium or ethanolamine salt.

Additionally, the invention relates to the use of a pharmaceutical composition containing at least one compound of trans-4-[4-(3-chloro-4-methoxybenzylamino)-benzothieno-[2,3-d]pyrimidin-2-yl]cyclohexane-carboxylic acid or a physiologically acceptable salt thereof together with at least one solid, liquid or semiliquid excipient or adjunct for the treatment of extrapyramidal movement disorders chosen from the group consisting of Gilles de la Tourette syndrome, ballism, myoclonus, restless legs syndrome and Wilson's disease.

The extrapyramidal movement disorders such as Steele-Richardson-Olszewski syndrome (= progressive supranuclear palsy), cortico-basal degeneration, olivo-ponto cerebellar atrophy, Shy Drager syndrome, minor chorea, chorea of pregnancy, writer's cramp, blepharospasm, Meige syndrome, dopa-sensitive dystonia, Gilles de la Tourette syndrome, ballism, myoclonus, restless legs syndrome, and Wilson's disease are not frequent enough to perform regular double-blind trials. However, the medical need in this field is pressing since no sufficient therapies are available so far.
Therefore, open-label observations in few selected patients are an adequate method to demonstrate the efficacy of trans-4-[4-(3-chloro-4-methoxybenzylamino)-benzothieno-[2,3-d]pyrimidin-2-yl]cyclohexane-carboxylic acid or a physiologically acceptable salt thereof.

All the pharmaceutical preparations used for the treatment of extrapyramidal movement disorders and/or for the treatment of adverse effects of anti-Parkinsonian drugs in extrapyramidal movement disorders including the medicinal combination can be used as pharmaceuticals in human or veterinary medicine.

### Pharmaceutical Salts and Other Forms

Trans-4-[4-(3-chloro-4-methoxybenzylamino)-benzothieno-[2,3-d]pyrimidin-2-yl]cyclohexanecarboxylic acid may be utilized in their final, non-salt form. On the other hand, it is also within the scope of the present invention to utilize the compound in the form of its pharmaceutically acceptable salts derived from various organic bases in accordance with procedures well known in the art. Pharmaceutically acceptable salt forms of trans-4-[4-(3-chloro-4-methoxybenzylamino)-benzothieno-[2,3-d]pyrimidin-2-yl]cyclo-hexanecarboxylic acid are prepared for the most part by conventional means. A suitable salt may be formed by reacting the compound with an appropriate base to provide the corresponding base addition salt. Examples of such bases are alkali metal hydroxides including potassium hydroxide, sodium hydroxide, and lithium hydroxide; alkaline earth metal hydroxides such as barium hydroxide and calcium hydroxide; alkali metal alkoxides, e.g., potassium ethanolate and sodium propanolate; and various organic bases such as piperidine, diethanolamine, and N-methylglutamine. Also included are the aluminum salts of trans-4-[4-(3-chloro-4-methoxybenzylamino)-benzothieno-[2,3-d]pyrimidin-2-yl]cyclohexane-carboxylic acid.
Further, base salts of trans-4-[4-(3-chloro-4-methoxybenzylamino)-benzothieno-[2,3-d]pyrimidin-2-yl]cyclohexanecarboxylic acid include, but are not limited to aluminum, ammonium, calcium, copper, ferric, ferrous, lithium, magnesium, manganic, manganous, potassium, sodium, and zinc salts. Preferred among the above recited salts are ammonium; the alkali metal salts sodium and potassium; and the alkaline earth metal salts calcium and magnesium. Salts of trans-4-[4-(3-chloro-4-methoxybenzyl-amino)-benzothieno-[2,3-d]pyrimidin-2-yl]cyclohexanecarboxylic acid derived from pharmaceutically acceptable organic non-toxic bases include, but are not limited to salts of primary, secondary, and tertiary' amines, substituted amines including naturally occurring substituted amines, cyclic amines, and basic ion exchange resins, e.g., arginine, betaine, caffeine, chloroprocaine, choline, N,N'-dibenzylethylenediamine (benzathine), dicyclohexylamine, diethanolamine, diethylamine, 2-diethylaminoethanol, 2-dimethylaminoethanol, ethanolamine, ethylenediamine, N-ethylmorpholine, N- ethylpiperidine, glucamine, glucosamine, histidine, hydrabamine, isopropylamine, lidocaine, lysine, meglumine, N-methyl-D-glucamine, morpholine, piperazine, piperidine, polyamine resins, procaine, purines, theobromine, triethanolamine, triethylamine, trimethylamine, tripropylamine, and tris-(hydroxymethyl)-methylamine (tromethamine).

As indicated, the pharmaceutically acceptable base addition salts of trans-4-[4-(3-chloro-4-methoxybenzylamino)-benzothieno-[2,3-d]pyrimidin-2-yl]cyclohexanecarboxylic acid are formed with metals or amines, such as alkali metals and alkaline earth metals, or organic amines. Preferred metals are sodium, potassium, magnesium, and calcium. Preferred organic amines are N,N'-dibenzylethylenediamine, chloroprocaine, choline, diethanolamine, ethylenediamine, N-methyl-D-glucamine, and procaine.

The base addition salts of trans-4-[4-(3-chloro-4-methoxybenzylamino)-benzothieno-[2,3-d]pyrimidin-2-yl]cyclohexanecarboxylic acid are prepared by contacting the free acid form with a sufficient amount of the desired base to produce the salt in the conventional manner. The free acid form may be regenerated by contacting the salt form with an acid and isolating the free acid form in the conventional manner. The free acid forms differ from their respective salt forms somewhat in physical properties such as solubility in polar solvents, but otherwise the salts are equivalent to their respective free acid form for purposes of the present invention.

In light of the above, it can be seen that the expression "pharmaceutically acceptable salt" as used herein is intended to mean an active ingredient comprising trans-4-[4-(3-chloro-4-methoxybenzylamino)-benzothieno-[2,3-d]pyrimidin-2-yl]cyclohexanecarboxylic acid utilized in the form of a salt thereof, especially where said salt form confers on said active ingredient improved pharmacokinetic properties as compared to the free form of said active ingredient or some other salt form of said active ingredient utilized previously. The pharmaceutically acceptable salt form of said active ingredient may also initially confer a desirable pharmacokinetic property on said active ingredient which it did not previously possess, and may even positively affect the pharmacodynamics of said active ingredient with respect to its therapeutic activity in the body.

The pharmacokinetic properties of said active ingredient which may be favorably affected include, e.g., the manner in which said active ingredient is transported across cell membranes, which in turn may directly and positively affect the absorption, distribution, biotransformation and excretion of said active ingredient. While the route of administration of the pharmaceutical composition is important, and various anatomical, physiological and pathological factors can critically affect bioavailability, the solubility of said active ingredient is usually dependent upon the character of the particular salt form thereof which it utilized. Further, as the artisan will appreciate, an aqueous solution of said active ingredient will provide the most rapid absorption of said active ingredient into the body of a patient being treated, while lipid solutions and suspensions, as well as solid dosage forms, will result in less rapid absorption of said active ingredient.

Oral ingestion of an active ingredient is the most preferred route of administration for reasons of safety, convenience, and economy, but absorption of such an oral dosage form can be adversely affected by physical characteristics such as polarity, emesis caused by irritation of the gastrointestinal mucosa, destruction by digestive enzymes and low pH, irregular absorption or propulsion in the presence of food or other drugs, and metabolism by enzymes of the mucosa, the intestinal flora, or the liver. Formulation of said active ingredient into different pharmaceutically acceptable salt forms may be effective in overcoming or alleviating one or more of the above-recited problems encountered with absorption of oral dosage forms.

### Pharmaceutical Compositions and Formulations

The description which follows concerns the manner in which trans-4-[4-(3-chloro-4-methoxybenzylamino)-benzothieno-[2,3-d]pyrimidin-2-yl]cyclo-hexanecarboxylic acid together with other therapeutic agents or non-therapeutic agents where these are desired, are combined with what are for the most part conventional pharmaceutically acceptable carriers to form dosage forms suitable for the different routes of administration which are utilized for any given patient, as well as appropriate to the disease, disorder, or condition for which any given patient is being treated.

The pharmaceutical compositions of the present invention comprise trans-4-[4-(3-chloro-4-methoxybenzylamino)-benzothieno-[2,3-d]pyrimidin-2-yl]cyclohexanecarboxylic acid or a pharmaceutically acceptable salt thereof as also above-described, together with a pharmaceutically acceptable carrier in accordance with the properties and expected performance of such carriers which are well-known in the pertinent art.

The amount of active ingredient that may be combined with the carrier materials to produce a single dosage form will vary depending upon the host treated, and the particular mode of administration. It should be understood, however, that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of active ingredient may also depend upon the therapeutic or prophylactic agent, if any, with which the ingredient is co-administered.

It is within the scope of the present invention to utilize trans-4-[4-(3-chloro-4-methoxybenzylamino)-benzothieno-[2,3-d]pyrimidin-2-yl]cyclohexanecarboxylic acid in the form of pharmaceutically acceptable salts derived from various organic and inorganic bases. An active ingredient comprising a preferred compound is often utilized in the form of a salt thereof, especially where said salt form confers on said active ingredient improved pharmacokinetic properties as compared to the free form of said active ingredient or some other salt form of said active ingredient utilized previously. The pharmaceutically acceptable salt form of said active ingredient may also initially confer a desirable pharmacokinetic property on said active ingredient which it did not previously possess, and may even positively affect the pharmacodynamics of said active ingredient with respect to its therapeutic activity in the body.

The pharmacokinetic properties of said active ingredient which may be favorably affected include, e.g., the manner in which said active ingredient is transported across cell membranes, which in turn may directly and positively affect the absorption, distribution, biotransformation and excretion of said active ingredient. While the route of administration of the pharmaceutical composition is important, and various anatomical, physiological and pathological factors can critically affect bioavailability, the solubility of said active ingredient is usually dependent upon the character of the particular salt form thereof which it utilized. Further, as the artisan understands, an aqueous solution of said active ingredient will provide the most rapid absorption of said active ingredient into the body of a patient being treated, while lipid solutions and suspensions, as well as solid dosage forms, will result in less rapid absorption of said active ingredient. Oral ingestion of said active ingredient is the most preferred route of administration for reasons of safety, convenience, and economy, but absorption of such an oral dosage form can be adversely affected by physical characteristics such as polarity, emesis caused by irritation of the gastrointestinal mucosa, destruction by digestive enzymes and low pH, irregular absorption or propulsion in the presence of food or other drugs, and metabolism by enzymes of the mucosa, the intestinal flora, or the liver. Formulation of said active ingredient into different harmaceutically acceptable salt forms may be effective in overcoming or alleviating one or more of the above- recited problems encountered with absorption of oral dosage forms.

The pharmaceutical compositions of the present invention comprise trans-4-[4-(3-chloro-4-methoxybenzylamino)-benzothieno-[2,3-d]pyrimidin-2-yl]cyclohexanecarboxylic acid or a pharmaceutically acceptable salt thereof as also above-described, together with a pharmaceutically acceptable carrier in accordance with the properties and expected performance of such carriers which are well-known in the pertinent art.

The term "carrier" as used herein includes acceptable diluents, excipients, adjuvants, vehicles, solubilization aids, viscosity modifiers, preservatives and other agents well known to the artisan for providing favorable properties in the final pharmaceutical composition. In order to illustrate such carriers, there follows a brief survey of pharmaceutically acceptable carriers that may be used in the pharmaceutical compositions of the present invention, and thereafter a more detailed description of the various types of ingredients. Typical carriers include but are by no means limited to, ion exchange compositions; alumina; aluminum stearate; lecithin; serum proteins, e.g., human serum albumin; phosphates; glycine; sorbic acid; potassium sorbate; partial glyceride mixtures of saturated vegetable fatty acids; hydrogenated palm oils; water; salts or electrolytes, e.g., prolamine sulfate, disodium hydrogen phosphate, potassium hydrogen phosphate, sodium chloride, and zinc salts; colloidal silica; magnesium trisilicate; polyvinyl pyrrolidone; cellulose-based substances; e.g., sodium carboxy-methylcellulose; polyethylene glycol; polyacrylates; waxes; polyethylene-polyoxypropylene-block polymers; and wool fat.

More particularly, the carriers used in the pharmaceutical compositions of the present invention comprise various classes and species of additives which are members independently selected from the groups consisting essentially of those recited in the following paragraphs.

Acidifying and alkalizing agents are added to obtain a desired or predetermined pH and comprise acidifying agents, e.g., acetic acid, glacial acetic acid, malic acid, and propionic acid. Stronger acids such as hydrochloric acid, nitric acid and sulfuric acid may be used but are less preferred. Alkalizing agents include, e.g., edetol, potassium carbonate, potassium hydroxide, sodium borate, sodium carbonate, and sodium hydroxide. Alkalizing agents which contain active amine groups, such as diethanolamine and trolamine, may also be used.

Aerosol propellants are required where the pharmaceutical composition is to be delivered as an aerosol under significant pressure. Such propellants include, e.g., acceptable fluorochlorohydrocarbons such as dichlorodifluoromethane, dichlorotetrafluoroethane, and trichloromonofluoromethane; nitrogen; or a volatile hydrocarbon such as butane, propane, isobutane or mixtures thereof.

Antimicrobial agents including antibacterial, antifungal and antiprotozoal agents are added where the pharmaceutical composition is topically applied to areas of the skin which are likely to have suffered adverse conditions or sustained abrasions or cuts which expose the skin to infection by bacteria, fungi or protozoa. Antimicrobial agents include such compounds as benzyl alcohol, chlorobutanol, phenylethyl alcohol, phenyl-mercuric acetate, potassium sorbate, and sorbic acid. Antifungal agents include such compounds as benzoic acid, butylparaben, ethylparaben, methylparaben, propylparaben, and sodium benzoate.

Antimicrobial preservatives are added to the pharmaceutical compositions of the present invention in order to protect them against the growth of potentially harmful microorganisms, which usually invade the aqueous phase, but in some cases can also grow in the oil phase of a composition. Thus, preservatives with both aqueous and lipid solubility are desirable. Suitable antimicrobial preservatives include, e.g., alkyl esters of p-hydroxybenzoic acid, propionate salts, phenoxyethanol, methylparaben sodium, propylparaben sodium, sodium dehydroacetate, benzalkonium chloride, benzethonium chloride, benzyl alcohol, hydantoin derivatives, quaternary ammonium compounds and cationic polymers, imidazolidinyl urea, diazolidinyl urea, and trisodium ethylenediamine tetracetate (EDTA). Preservatives, are preferably employed in amounts ranging from about 0.01 % to about 2.0% by weight of the total composition.

Antioxidants are added to protect all of the ingredients of the pharmaceutical composition from damage or degradation by oxidizing agents present in the composition itself or the use environment, e.g., anoxomer, ascorbyl palmitate, butylated hydroxyanisole, butylated hydroxytoluene, hypophosphorous acid, potassium metabisulfite, propyl octyl and dodecyl gallate, sodium metabisulfite, sulfur dioxide, and tocopherols.

Buffering agents are used to maintain a desired pH of a composition once established, from the effects of outside agents and shifting equilibria of components of the composition. The buffering may be selected from among those familiar to the artisan skilled in the preparation of pharmaceutical compositions, e. g., calcium, acetate, potassium metaphosphate, potassium phosphate monobasic, and tartaric acid.

Chelating agents are used to help maintain the ionic strength of the pharmaceutical composition and bind to and effectively remove destructive compounds and metals, and include, e.g., edetate dipotassium, edetate disodium, and edetic acid.

Dermatologically active agents are added to the pharmaceutical compositions of the present invention where they are to be applied topically, and include, e.g., wound healing agents such as peptide derivatives, yeast, panthenol, hexylresorcinol, phenol, tetracycline hydrochloride, lamin and kinetin; retinoids for treating skin cancer, e.g., retinol, tretinoin, isotretinoin, etretinate, acitretin, and arotinoid; mild antibacterial agents for treating skin infections, e.g., resorcinol, salicylic acid, benzoyl peroxide, erythromycin-benzoyl peroxide, erythromycin, and clindamycin; antifungal agents for treating tinea corporis, tinea pedis, candidiasis and tinea versicolor, e.g., griseofulvin, azoles such as miconazole, econazole, itraconazole, fluconazole, and ketoconazole, and allylamines such as naftifine and terfinafine; antiviral agents for treating cutaneous herpes simplex, herpes zoster, and chickenpox, e.g., acyclovir, famciclovir, and valacyclovir; antihistamines for treating pruritis, atopic and contact dermatitis, e.g., diphenhydramine, terfenadine, asternizole, loratadine, cetirizi, ne, acrivastine, and temelastine; topical anesthetics for relieving pain, irritation and itching, e.g., benzocaine, lidocaine, dibucaine, and pramoxine hydrochloride; topical analgesics for relieving pain and inflammation, e.g., methyl salicylate, camphor, menthol, and resorcinol; topical antiseptics for preventing infection, e.g., benzalkonium chloride and povidone-iodine; and vitamins and derivatives thereof such as tocopherol, tocopherol acetate, retinoic acid and retinol.

Dispersing and suspending agents are used as aids for the preparation of stable formulations and include, e.g., poligeenan, povidone, and silicon dioxide.

Emollients are agents, preferably non-oily and water-soluble, which soften and soothe the skin, especially skin that has become dry because of excessive loss of water. Such agents are used with pharmaceutical compositions of the present invention which are intended for topical applications, and include, e.g., hydrocarbon oils and waxes, triglyceride esters, acetylated monoglycerides, methyl and other alkyl esters of C₁₀-C₂₀ fatty acids, C₁₀ - C₂₀ fatty acids, C₁₀ -C₂₀ fatty alcohols, lanolin and derivatives, polyhydric alcohol esters such as polyethylene glycol (200-600), polyoxyethylene sorbitan fatty acid esters, wax esters, phospholipids, and sterols; emulsifying agents used for preparing oil-in-water emulsions; excipients, e.g., laurocapram and polyethylene glycol monomethyl ether; humectants, e.g., sorbitol, glycerin and hyaluronic acid; ointment bases, e.g., petrolatum, polyethylene glycol, lanolin, and poloxamer; penetration enhancers, e.g., dimethyl isosorbide, diethyl-glycol monoethylether, 1-dodecylazacycloheptan-2-one, and dimethylsulfoxide (DMSO); preservatives, e.g., benzalkonium chloride, benzethonium chloride, alkyl esters of p- hydroxybenzoic acid, hydantoin derivatives, cetylpyridinium chloride, propylparaben, quaternary ammonium compounds such as potassium benzoate, and thimerosal; sequestering agents comprising cyclodextrins; solvents, e.g., acetone, alcohol, amylene hydrate, butyl alcohol, corn oil, cottonseed oil, ethyl acetate, glycerin, hexylene glycol, isopropyl alcohol, isostearyl alcohol, methyl alcohol, methylene chloride, mineral oil, peanut oil, phosphoric acid, polyethylene glycol, polyoxypropylene 15 stearyl ether, propylene glycol, propylene glycol diacetate, sesame oil, and purified water; stabilizers, e.g., calcium saccharate and thymol; surfactants, e.g., lapyrium chloride; laureth 4, ie., α-dodecyl-ω-hydroxy-poly(oxy-1,2-ethanediyl) or polyethylene glycol monododecyl ether.

Emulsifying agents, including emulsifying and stiffening agents and emulsion adjuncts, are used for preparing oil-in-water emulsions when these form the basis of the pharmaceutical compositions of the present invention. Such emulsifying agents include, e.g., non-ionic emulsifiers such as C₁₀ -C₂₀ fatty alcohols and said fatty alcohols condensed with from 2 to 20 moles of ethylene oxide or propylene oxide, (C₆ -C₁₂)alkyl phenols condensed with from 2 to 20 moles of ethylene oxide, mono- and di-C₁₀-C₂₀ fatty acid esters of ethylene glycol, C₁₀ -C₂₀ fatty acid monoglyceride, diethylene glycol, polyethylene glycols of MW 200 6000, polypropylene glycols of MW 200-3000, and particularly sorbitol, sorbitan, polyoxyethylene sorbitol, polyoxyethylene sorbitan, hydrophilic wax esters, cetostearyl alcohol, oleyl alcohol, lanolin alcohols, cholesterol, mono- and di-glycerides, glyceryl monostearate, polyethylene glycol monostearate, mixed mono- and distearic esters of ethylene glycol and polyoxyethylene glycol, propylene glycol monostearate, and hydroxypropyl cellulose. Emulsifying agents which contain active amine groups may also be used and typically include anionic emulsifiers such as fatty acid soaps, e.g., sodium, potassium and triethanolamine soaps of C₁₀ -C₂₀ fatty acids; alkali metal, ammonium or substituted ammonium (C₁₀ -C₃₀)alkyl sulfates, (C₁₀ -C₃₀)alkyl sulfonates, and (C₁₀ -C₅₀)alkyl ethoxy ether sulfonates. Other suitable emulsifying agents include castor oil and hydrogenated castor oil; lecithin; and polymers of 2-propenoic acid together with polymers of acrylic acid, both cross-linked with allyl ethers of sucrose and/or pentaerythritol, having varying viscosities and identified by product names carbomer 910, 934, 934P, 940, 941, and 1342. Cationic emulsifiers having active amine groups may also be used, including those based on quaternary ammonium, morpholinium and pyridinium compounds. Similarly, amphoteric emulsifiers having active amine groups, such as cocobetaines, lauryl dimethylamine oxide and cocoylimidazoline, may be used. Useful emulsifying and stiffening agents also include cetyl alcohol and sodium stearate; and emulsion adjuncts such as oleic acid, stearic acid, and stearyl alcohol.

Excipients include, e.g., laurocapram and polyethylene glycol monomethyl ether.
Where the pharmaceutical composition of the present invention is to be applied topically, penetration enhancers may be used, which include, e.g., dimethyl isosorbide, diethyl-glycol-monoethylether, 1-dodecylazacyclo-heptan-2-one, and dimethylsulfoxide (DMSO). Such compositions will also typically include ointment bases, e.g., petrolatum, polyethylene glycol, lanolin, and poloxamer, which is a block copolymer of polyoxyethylene and polyoxypropylene, which may also serve as a surfactant or emulsifying agent.

Preservatives are used to protect pharmaceutical compositions of the present invention from degradative attack by ambient microorganisms, and include, e.g., benzalkonium chloride, benzethonium chloride, alkyl esters of p-hydroxybenzoic acid, hydantoin derivatives, cetylpyridinium chloride, monothioglycerol, phenol, phenoxyethanol, methylparagen, imidazolidinyl urea, sodium dehydroacetate, propylparaben, quaternary ammonium compounds, especially polymers such as polixetonium chloride, potassium benzoate, sodium formaldehyde sulfoxylate, sodium propionate, and thimerosal.

Sequestering agents are used to improve the stability of the pharmaceutical compositions of the present invention and include, e.g., the cyclodextrins which are a family of natural cyclic oligosaccharides capable of forming inclusion complexes with a variety of materials, and are of varying ring sizes, those having 6-, 7- and 8-glucose residues in a ring being commonly referred to as α-cyclodextrins, β-cyclodextrins, and γ-cyclodextrins, respectively. Suitable cyclodextrins include, e.g., α-cyclodextrin, β-cyclodextrin, γ-cyclodextrin, δ-cyclodextrin and cationized cyclodextrins.

Solvents which may be used in preparing the pharmaceutical compositions of the present invention include, e.g., acetone, alcohol, amylene hydrate, butyl alcohol, corn oil, cottonseed oil, ethyl acetate, glycerin, hexylene glycol, isopropyl alcohol, isostearyl alcohol, methyl alcohol, methylene chloride, mineral oil, peanut oil, phosphoric acid, polyethylene glycol, polyoxypropylene 15 stearyl ether, propylene glycol, propylene glycol diacetate, sesame oil, and purified water.

Stabilizers which are suitable for use include, e.g., calcium saccharate and thymol.
Stiffening agents are typically used in formulations for topical applications in order to provide desired viscosity and handling characteristics and include, e.g., cetyl esters wax, myristyl alcohol, parafin, synthetic parafin, emulsifying wax, microcrystalline wax, white wax and yellow wax.

Sugars are often used to impart a variety of desired characteristics to the pharmaceutical compositions of the present invention and in order to improve the results obtained, and include, e.g., monosaccharides, disaccharides and polysaccharides such as glucose, xylose, fructose, reose, ribose, pentose, arabinose, allose, tallose, altrose, mannose, galactose, lactose, sucrose, erythrose, glyceraldehyde, or any combination thereof.

Surfactants are employed to provide stability for multi-component pharmaceutical compositions of the present invention, enhance existing properties of those compositions, and bestow desirable new characteristics on said compositions. Surfactants are used as wetting agents, antifoam agents, for reducing the surface tension of water, and as emulsifiers, dispersing agents and penetrants, and include, e.g., lapyrium chloride; laureth 4, i.e., α-dodecyl-ω-hydroxy-poly(oxy-1,2-ethanediyl) or polyethylene glycol monododecyl ether; laureth 9, i.e., a mixture of polyethylene glycol monododecyl ethers averaging about 9 ethylene oxide groups per molecule; monoethanolamine; nonoxynol 4, 9 and 10, i.e., polyethylene glycol mono(p-nonylphenyl) ether; nonoxynol 15, i. e., α-(p-nonylphenyl)-ω-hydroxypenta-deca(oxyethylene); nonoxynol 30, i.e. , α-(p-nonylphenyl)-ω-hydroxytriaconta(oxyethylene); poloxalene, i.e., nonionic polymer of the polyethylenepolypropylene glycol type, MW = approx. 3000; poloxamer, referred to in the discussion of ointment bases further above; polyoxyl 8, 40 and 50 stearate, i.e., poly(oxy-1,2-ethanediyl), α-hydro-ω-hydroxy-octa-decanoate; polyoxyl 10 oleyl ether, i.e., poly(oxy-1,2-ethanediyl), α-[(Z)-9-octadecenyl-ω-hydroxy-; polysorbate 20, i.e., sorbitan, monododecanoate, poly(oxy-1,2-ethanediyl); polysorbate 40, i.e., sorbitan, monohexadecanoate, poly(oxy-1,2-ethanediyl); polysorbate 60, i.e., sorbitan, monooctadecanoate, poly(oxy-1,2-ethanediyl); polysorbate 65, i.e., sorbitan, trioctadecanoate, poly(oxy-1,2-ethanediyl); polysorbate 80, i.e., sorbitan, mono-9 -monodecenoate, poly(oxy-1,2-ethanediyl); polysorbate 85, i.e., sorbitan, tri-9-octadecenoate, poly(oxy-1,2-ethanediyl); sodium lauryl sulfate; sorbitan monolaurate; sorbitan monooleate; sorbitan monopalmitate; sorbitan monostearate; sorbitan sesquioleate; sorbitan trioleate; and sorbitan tristearate.

The pharmaceutical compositions of the present invention may be prepared using very straightforward methodology which is well understood by the artisan of ordinary skill. Where the pharmaceutical compositions of the present invention are simple aqueous and/or other solvent solutions, the various components of the overall composition are brought together in any practical order, which will be dictated largely by considerations of convenience. Those components having reduced water solubility, but sufficient solubility in the same co-solvent with water, may all be dissolved in said co-solvent, after which the co- solvent solution will be added to the water portion of the carrier whereupon the solutes therein will become dissolved in the water. To aid in this dispersion/solution process, a surfactant may be employed.

Where the pharmaceutical compositions of the present invention are to be in the form of emulsions, the components of the pharmaceutical composition will be brought together in accordance with the following general procedures. The continuous water phase is first heated to a temperature in the range of from about 60° to about 95°C, preferably from about 70° to about 85°C, the choice of which temperature to use being dependent upon the physical and chemical properties of the components which make up the oil-in-water emulsion. Once the continuous water phase has reached its selected temperature, the components of the final composition to be added at this stage are admixed with the water and dispersed therein under high-speed agitation. Next, the temperature of the water is restored to approximately its original level, after which the components of the composition which comprise the next stage are added to the composition mixture under moderate agitation and mixing continues for from about 5 to about 60 minutes, preferably about 10 to about 30 minutes, depending on the components of the first two stages. Thereafter, the composition mixture is passively or actively cooled to from about 20° to about 55°C for addition of any components in the remaining stages, after which water is added in sufficient quantity to reach its original predetermined concentration in the overall composition.

According to the present invention, the pharmaceutical compositions may be in the form of a sterile injectable preparation, for example a sterile injectable aqueous or oleaginous suspension. This suspension may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3- butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as do natural pharmaceutically acceptable oils, such as olive oil or castor oil, especially in their polyoxyethylated versions. These oil solutions or suspensions may also contain a long-chain alcohol diluent or dispersant, such as Rh, HClX or similar alcohol.

The pharmaceutical compositions of the present invention may be orally administered in any orally acceptable dosage form including, but not limited to, capsules, tablets, aqueous suspensions or solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending agents. If desired, certain sweetening, flavoring or coloring agents may also be added. Alternatively, the pharmaceutical compositions of this invention may be administered in the form of suppositories for rectal administration. These can be prepared by mixing the agent with a suitable non-irritating excipient which is solid at room temperature but liquid at the rectal temperature and therefore will melt in the rectum to release the drug. Such materials include cocoa butter, beeswax and polyethylene glycols.

The pharmaceutical compositions of the present invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation, as described above, or in a suitable enema formulation. Topically active transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate, cetyl esters wax; cetearyl alcohol, 2- octyldodecanol, benzyl alcohol and water.

Pharmaceutical compositions within the scope of the present invention include those wherein the therapeutically effective amount of an active ingredient comprising trans-4-[4-(3-chloro-4-methoxybenzylamino)-benzothieno-[2,3-d]pyrimidin-2-yl]cyclohexanecarboxylic acid required for treating or preventing diseases, disorders, and conditions as described herein, is provided in a dosage form suitable for systemic administration.

Such a pharmaceutical composition will contain said active ingredient in suitable liquid form for delivery by: (1) injection or infusion which is intraarterial, intra- or transdermal, subcutaneous, intramuscular, intraspinal, intrathecal, or intravenous, wherein said active ingredient: (a) is contained in solution as a solute; (b) is contained in the discontinuous phase of an emulsion, or the discontinuous phase of an inverse emulsion which inverts upon injection or infusion, said emulsions containing suitable emulsifying agents; or (c) is contained in a suspension as a suspended solid in colloidal or microparticulate form, said suspension containing suitable suspending agents; (2) injection or infusion into suitable body tissues or cavities as a depot, wherein said composition provides storage of said active ingredient and thereafter delayed-, sustained-, and/or controlled-release of said active ingredient for systemic distribution; (3) instillation, inhalation or insufflation into suitable body tissues or cavities of said pharmaceutical composition in suitable solid form, where said active ingredient: (a) is contained in a solid implant composition providing delayed-, sustained-, and/or controlled-release of said active ingredient; (b) is contained in a particulate composition to be inhaled into the lungs; or (c) is contained in a particulate composition to be blown into suitable body tissues or cavities, where said composition optionally provides delayed-, sustained-, and/or controlled-release of said active ingredient; or (4) ingestion of said pharmaceutical composition in suitable solid or liquid form for peroral delivery of said active ingredient, where said active ingredient is contained in a solid dosage form; or (b) is contained in a liquid dosage form.

Particular dosage forms of the above-described pharmaceutical compositions include (1) suppositories as a special type of implant, comprising bases which are solid at room temperature but melt at body temperature, slowly releasing the active ingredient with which they are impregnated into the surrounding tissue of the body, where the active ingredient becomes absorbed and transported to effect systemic administration; (2) solid peroral dosage forms selected from the group consisting of (a) delayed-release oral tablets, capsules, caplets, lozenges, troches, and multiparticulates; (b) enteric-coated tablets and capsules which prevent release and absorption in the stomach to facilitate delivery distal to the stomach of the patient being treated; (c) sustained-release oral tablets, capsules and microparticulates which provide systemic delivery of the active ingredient in a controlled manner up to a 24-hour period; (d) fast-dissolving tablets; (e) encapsulated solutions; (f) an oral paste; (g) a granular form incorporated in or to be incorporated in the food of a patient being treated; and (h) liquid peroral dosage forms selected from the group consisting of solutions, suspensions, emulsions, inverse emulsions, elixirs, extracts, tinctures, and concentrates.

Pharmaceutical compositions within the scope of the present invention include those wherein the therapeutically effective amount of an active ingredient comprising trans-4-[4-(3-chloro-4-methoxybenzylamino)-benzothieno-[2,3-d]pyrimidin-2-yl]cyclohexanecarboxylic acid required for treating or preventing diseases, disorders, and conditions as described herein is provided in a dosage form suitable for local administration to a patient being treated, wherein said pharmaceutical composition contains said active ingredient in suitable liquid form for delivering said active ingredient by: (1) injection or infusion into a local site which is intraarterial, intraarticular, intrachondrial, intracostal, intracystic, intra- or transdermal, intrafasicular, intraligamentous, intramedulary, intramuscular, intranasal, intraneural, intraocular, i.e., opthalmic administration, intraosteal, intrapelvic, intrapericardial, intraspinal, intrasternal, intrasynovial, intratarsal, or intrathecal; including components which provide delayed-release, controlled-release, and/or sustained-release of said active ingredient into said local site; where said active ingredient is contained: (a) in solution as a solute; (b) in the discontinuous phase of an emulsion, or the discontinuous phase of an inverse emulsion which inverts upon injection or infusion, said emulsions containing suitable emulsifying agents; or (c) in a suspension as a suspended solid in colloidal or microparticulate form, said suspension containing suitable suspending agents; or (2) injection or infusion as a depot for delivering said active ingredient to said local site; wherein said composition provides storage of said active ingredient and thereafter delayed-, sustained-, and/or controlled- release of said active ingredient into said local site, and wherein said composition also includes components which ensure that said active ingredient has predominantly local activity, with little systemic carryover activity; or wherein said pharmaceutical composition contains said active ingredient in suitable solid form for delivering said inhibitor by: (3) instillation, inhalation or insufflation to said local site, where said active ingredient is contained: (a) in a solid implant composition which is installed in said local site, said composition optionally providing delayed-, sustained-, and/or controlled-release of said active ingredient to said local site; (b) in a particulate composition which is inhaled into a local site comprising the lungs; or (c) in a particulate composition which is blown into a local site, where said composition includes components which will ensure that said active ingredient has predominantly local activity, with insignificant sys temic carryover activity, and optionally provides delayed-, sustained- , and/or controlled release of said active ingredient to said local site. For ophthalmic use, the pharmaceutical compositions may be formulated as micronized suspension in isotonic, pH adjusted sterile saline, or, preferably, as solutions in isotonic, pH adjusted sterile saline, either with our without a preservative such as benzylalkonium chloride. Alternatively, for ophthalmic uses, the pharmaceutical compositions may be formulated in an ointment such as petrolatum.

The pharmaceutical compositions of the present invention may also be administered by nasal aerosol or inhalation through the use of a nebulizer, a dry powder inhaler or a metered dose inhaler. Such compositions are prepared according to techniques well-known in the art of pharmaceutical formulation and may be prepared as solutions in saline, employing benzyl alcohol or other suitable preservatives, absorption promoters to enhance bioavailability, hydrofluorocarbons, and/or other conventional solubilizing or dispersing agents.

As already mentioned, trans-4-[4-(3-chloro-4-methoxybenzylamino)-benzothieno-[2,3-d]pyrimidin-2-yl]cyclohexanecarboxylic acid may be administered systemically to a patient to be treated as a pharmaceutical composition in suitable liquid form by injection or infusion. There are a number of sites and organ systems in the body of the patient which will allow the properly formulated pharmaceutical composition, once injected or infused, to permeate the entire body and all of the organ system of the patient being treated. An injection is a single dose of the pharmaceutical composition forced, usually by a syringe, into the tissue involved. The most common types of injections are intramuscular, intravenous, and subcutaneous. By contrast, an infusion is the gradual introduction of the pharmaceutical composition into the tissue involved. The most common type of infusion is intravenous. Other types of injection or infusion comprise intraarterial, intra- or transdermal (including subcutaneous), or intraspinal especially intrathecal. In these liquid pharmaceutical compositions, the active ingredient may be contained in solution as the solute. This is the most common and most preferred type of such composition, but requires an active ingredient in a salt form that has reasonably good aqueous solubility. Water (or saline) is by far the most preferred solvent for such compositions. Occasionally supersaturated solutions may be utilized, but these present stability problems that make them impractical for use on an everyday basis.

If it is not possible to obtain a form of some preferred compound that has the requisite degree of aqueous solubility, as may sometimes occur, it is, within the skill of the artisan to prepare an emulsion, which is a dispersion of small globules of one liquid, the discontinuous or internal phase, throughout a second liquid, the continuous or external phase, with which it is immiscible. The two liquids are maintained in an emulsified state by the use of emulsifiers which are pharmaceutically acceptable. Thus, if the active ingredient is a waterinsoluble oil, it can be administered in, an emulsion of which it is the discontinuous phase. Also where the active ingredient is water-insoluble but can be dissolved in a solvent which is immiscible with water, an emulsion can be used. While the active ingredient would most commonly be used as the discontinuous or internal phase of what is referred to as an oil-in- water emulsion, it could also be used as the discontinuous or internal phase of an inverse emulsion, which is commonly referred to as a water-in- oil emulsion. Here the active ingredient is soluble in water and could be administered as a simple aqueous solution. However, inverse emulsions invert upon injection or infusion into an aqueous medium such as the blood, and offer the advantage of providing a more rapid and efficient dispersion of the active ingredient into that aqueous medium than can be obtained using an aqueous solution. Inverse emulsions are prepared by using suitable, pharmaceutically acceptable emulsifying agents well known in the art. Where the active ingredient has limited water solubility, it may also be administered as a suspended solid in colloidal or microparticulate form in a suspension prepared using suitable, pharmaceutically acceptable suspending agents. The suspended solids containing the active ingredient may also be formulated as delayed-, sustained-, and/or controlled-release compositions.

While systemic administration will most frequently be carried out by injection or infusion of a liquid, there are many situations in which it will be advantageous or even necessary to deliver the active ingredient as a solid. Systemic administration of solids is carried out by instillation, inhalation or insufflation of a pharmaceutical composition in suitable solid form containing the active ingredient. Instillation of the active ingredient may entail installing a solid implant composition into suitable body tissues or cavities. The implant may comprise a matrix of bio-compatible and bioerodible materials in which particles of a solid active ingredient are dispersed, or in which, possibly, globules or isolated cells of a liquid active ingredient are entrapped. Desirably, the matrix will be broken down and completely absorbed by the body. The composition of the matrix is also preferably selected to provide controlled-, sustained-, and/or delayed release of the active ingredient over extended periods of time, even as much as several months.

The term "implant" most often denotes a solid pharmaceutical composition containing the active ingredient, while the term "depot' usually implies a liquid pharmaceutical composition containing the active ingredient, which is deposited in any suitable body tissues or cavities to form a reservoir or pool which slowly migrates to surrounding tissues and organs and eventually becomes systemically distributed. However, these distinctions are not always rigidly adhered to in the art, and consequently, it is contemplated that there is included within the scope of the present invention liquid implants and solid depots, and even mixed solid and liquid forms for each. Suppositories may be regarded as a type of implant, since they comprise bases which are solid at room temperature but melt at a patient's body temperature, slowly releasing the active ingredient with which they are impregnated into the surrounding tissue of the patient's body, where the active ingredient becomes absorbed and transported to effect systemic administration.

Systemic administration can also be accomplished by inhalation or insufflation of a powder, i.e., particulate composition containing the active ingredient. For example, the active ingredient in powder form may be inhaled into the lungs using conventional devices for aerosolizing particulate formulations. The active ingredient as a particulate formulation may also be administered by insufflation, i.e., blown or otherwise dispersed into suitable body tissues or cavities by simple dusting or using conventional devices for aerosolizing particulate formulations. These particulate compositions may also be formulated to provide delayed-, sustained-, and/or controlled- release of the active ingredient in accordance with well understood principles and known materials.

Other means of systemic administration which may utilize the active ingredients of the present invention in either liquid or solid form include transdermal, intranasal, and opthalmic routes. In particular, transdermal patches prepared in accordance with well known drug delivery technology may be prepared and applied to the skin of a patient to be treated, whereafter the active- agent by reason of its formulated solubility characteristics migrates across the epidermis and info the dermal layers of the patient's skin where it is taken up as part of the general circulation of the patient, ultimately providing systemic distribution of the active ingredient over a desired, extended period of time. Also included are implants which are placed beneath the epidermal layer of the skin, i. e. between the epidermis and the dermis of the skin of the patient being treated. Such an implant will be formulated in accordance with well known principles and materials commonly used in this delivery technology, and may be prepared in such a way as to provide controlled-, sustained-, and/or delayed-release of the active ingredient into the systemic circulation of the patient. Such subepidermal (subcuticular) implants provide the same facility of installation and delivery efficiency as transdermal patches, but without the limitation of being subject to degradation, damage or accidental removal as a consequence of being exposed on the top layer of the patient's skin.

In the above description of pharmaceutical compositions containing a preferred compound, the equivalent expressions: "administration", "administration of', "administering", and "administering a" have been used with respect to said pharmaceutical compositions. As thus employed, these expressions are intended to mean providing to a patient in need of treatment a pharmaceutical composition of the present invention by any of the routes of administration herein described, wherein the active ingredient is a preferred compound or a prodrug, derivative, or metabolite thereof which is useful in treating a disease, disorder, or condition in said patient.

The dosage and dose rate of the compounds effective for treating or preventing,a disease, disorder, or condition will depend on a variety of factors, such as the nature of the inhibitor, the size of the patient, the goal of the treatment, the nature of the pathology to be treated, the specific pharmaceutical composition used, and the observations and conclusions of the treating physician.

For example, where the dosage form is oral, e.g., a tablet or capsule, suitable dosage levels of trans-4-[4-(3-chloro-4-methoxybenzylamino)-benzothieno-[2,3-d]pyrimidin-2-yl]cyclohexanecarboxylic acid or its salt will be between about 0.1 µg/kg and about 50.0 mg/kg of body weight per day, preferably between about 5.0 µg/kg and about 5.0 mg/kg of body weight per day, more preferably between about 10. 0 µg/kg and about 1.0 mg/kg of body weight per day, and most preferably between about 20.0 µg/kg and about 0.5 mg/kg of body weight per day of the active ingredient.

Where the dosage form is topically administered to the bronchia and lungs, e.g., by means of a powder inhaler or nebulizer, suitable dosage levels of the compounds will be between about 0.001 µg/kg and about 10.0 mg/kg of body weight per day, preferably between about 0.5 µg/kg and about 0.5 mg/kg of body weight per day, more preferably between about 1.0 µg/kg and about 0.1 mg/kg of body weight per day, and most preferably between about 2.0 µg/kg and about 0.05 mg/kg of body weight per day of the active ingredient.

Using representative body weights of 10 kg and 100 kg in order to illustrate the range of daily oral dosages which might be used as described above, suitable dosage levels of trans-4-[4-(3-chloro-4-methoxybenzylamino)-benzothieno-[2,3-d]pyrimidin-2-yl]cyclohexanecarboxylic acid will be between about 1.0 -10.0 µg and 500.0 - 5000.0 mg per day, preferably between about 50.0 - 500.0 µg and 50.0 - 500.0 mg per day, more preferably between about 100.0 - 1000.0 µg and 10.0 - 100.0 mg per day, and most perferably between about 200.0 - 2000.0 µg and about 5.0 - 50.0 mg per day of the active ingredient comprising a preferred compound. These ranges of dosage amounts represent total dosage amounts of the active ingredient per day for a given patient. The number of times per day that a dose is administered will depend upon such pharmacological and pharmacokinetic factors as the half-life of the active ingredient, which reflects its rate of catabolism and clearance, as well as the minimal and optimal blood plasma or other body fluid levels of said active ingredient attained in the patient which are required for therapeutic efficacy.

Numerous other factors must also be considered in deciding upon the number of doses per day and the amount of active ingredient per dose that will be administered. Not the least important of such other factors is the individual response of the patient being treated. Thus, for example, where the active ingredient is used to treat or prevent a disease, and is administered topically via aerosol inhalation into the lungs, from one to four doses consisting of acuations of a dispensing device, i.e., "puffs" of an inhaler, will be administered each day, each dose containing from about 50.0 µg to about 10.0 mg of active ingredient.

The examples which follow relate to pharmaceutical products:

### Example A: Vials

A solution of 100 g of trans-4-[4-(3-chloro-4-methoxybenzylamino)-benzothieno-[2,3-d]pyrimidin-2-yl]cyclohexanecarboxylic acid or a physiologically acceptable salt thereof and 5 g of disodium hydrogen phosphate in 3 l of twice-distilled water is brought to pH 6.5 with 2N hydrochloric acid, filter-sterilized, filled into vials, lyophilized under sterile conditions and sealed in sterile form. Each vial comprises 5 mg of active ingredient.

### Example B: Suppositories

A mixture of 20 g of trans-4-[4-(3-chloro-4-methoxybenzylamino)-benzothieno-[2,3-d]pyrimidin-2-yl]cyclohexanecarboxylic acid or a physiologically acceptable salt thereof is melted with 100 g of soya lecithin and 1400 g of cocoa butter, and the mixture is poured into moulds and left to cool. Each suppository comprises 20 mg of active ingredient.

### Example C: Solution

A solution is prepared from 1 g of trans-4-[4-(3-chloro-4-methoxybenzylamino)-benzothieno-[2,3-d]pyrimidin-2-yl]cyclohexane-carboxylic acid or a physiologically acceptable salt thereof, 9.38 g of NaH₂PO₄·2H₂O, 28.48 g of Na₂HPO₄·12H₂O and 0.1 g of benzalkonium chloride in 940 ml of twice-distilled water. The pH is brought to 6.8, and the solution is made up to 1 land sterilized by irradiation. This solution can be used in the form of eyedrops.

### Example D: Ointment

500 mg of trans-4-[4-(3-chloro-4-methoxybenzylamino)-benzothieno-[2,3-d]pyrimidin-2-yl]cyclohexanecarboxylic acid or a physiologically acceptable salt thereof are mixed with 99.5 g of petroleum jelly under aseptic conditions.

### Example E-1: Tablets

A mixture of 1 kg of trans-4-[4-(3-chloro-4-methoxybenzylamino)-benzothieno-[2,3-d]pyrimidin-2-yl]cyclohexanecarboxylic acid or a physiologically acceptable salt thereof, 4 kg of lactose, 1.2 kg of potato starch, 0.2 kg of talc and 0.1 kg of magnesium stearate is tableted in the customary manner in such a way that each tablet comprises 10 mg of active ingredient.

### Example E-2: Tablets

A mixture of 20 g of trans-4-[4-(3-chloro-4-methoxybenzylamino)-benzothieno-[2,3-d]pyrimidin-2-yl]cyclohexanecarboxylic acid sodium salt, 1 kg of I-dopa, 250 g benserazide, 4 kg of lactose, 1.6 kg of potato starch, 0.2 kg of talc and 0.1 kg of magnesium stearate is tableted in the customary manner in such a way that each tablet comprises 0,2 mg trans-4-[4-(3-chloro-4-methoxybenzylamino)-benzothieno-[2,3-d]pyrimidin-2-yl]cyclohexanecarboxylic acid sodium salt, 10 mg of I-dopa and 2,5 mg benserazide.

### Example F: Sugar-coated tablets

A mixture is tableted analogously to Example E, and the tablets are subsequently coated in the customary manner with a coating of sucrose, potato starch, talc, tragacanth and colouring.

### Example G: capsules

2 kg of trans-4-[4-(3-chloro-4-methoxybenzylamino)-benzothieno-[2,3-d]pyrimidin-2-yl]cyclohexanecarboxylic acid or a physiologically acceptable salt thereof are filled into hard gelatin capsules in the customary manner so that each capsule comprises 20 mg of the active ingredient.

### Example H: Ampoules

A solution of 1 kg of trans-4-[4-(3-chloro-4-methoxybenzylamino)-benzothieno-[2,3-d]pyrimidin-2-yl]cyclohexanecarboxylic acid or a physiologically acceptable salt thereof in 60 l of twice-distilled water is filter-sterilized, filled into ampoules, lyophilized under sterile conditions and sealed in sterile form. Each ampoule comprises 10 mg of active ingredient.

### Example I: Spray for inhalation

14 g of trans-4-[4-(3-chloro-4-methoxybenzylamino)-benzothieno-[2,3-d]pyrimidin-2-yl]cyclohexanecarboxylic acid or a physiologically acceptable salt thereof are dissolved in 10 l of isotonic NaCl solution, and the solution is filled into commercially available pump-operated spray containers. The solution can be sprayed into mouth or nose. One actuation (approximately 0.1 ml) corresponds to a dose of approximately 0.14 mg.

## Claims

1. Use of trans-4-[4-(3-chloro-4-methoxybenzylamino)-benzothieno-[2,3-d]pyrimidin-2-yl]cyclohexanecarboxylic acid or a physiologically acceptable salt thereof, for the manufacture of a medicament for the treatment of extrapyramidal movement disorders.

2. Use of trans-4-[4-(3-chloro-4-methoxybenzylamino)-benzothieno-[2,3-d]pyrimidin-2-yl]cyclohexanecarboxylic acid or a physiologically acceptable salt thereof, for the manufacture of a medicament for the treatment of adverse effects of anti-Parkinsonian drugs in idiopathic Parkinson's disease.

3. Use of trans-4-[4-(3-chloro-4-methoxybenzylamino)-benzothieno-[2,3-d]pyrimidin-2-yl]cyclohexanecarboxylic acid or a physiologically acceptable salt thereof, for the manufacture of a medicament for the treatment of idiopathic Parkinsons's disease.

4. Use of trans-4-[4-(3-chloro-4-methoxybenzylamino)-benzothieno-[2,3-d]pyrimidin-2-yl]cyclohexanecarboxylic acid or a physiologically acceptable salt thereof, for the manufacture of a medicament for the treatment of adverse effects of anti-Parkinsonian drugs in Parkinson's syndromes.

5. Use of trans-4-[4-(3-chloro-4-methoxybenzylamino)-benzothieno-[2,3-d]pyrimidin-2-yl]cyclohexanecarboxylic acid or a physiologically acceptable salt thereof, for the manufacture of a medicament for the treatment of Parkinsons syndromes.

6. Use of trans-4-[4-(3-chloro-4-methoxybenzylamino)-benzothieno-[2,3-d]pyrimidin-2-yl]cyclohexanecarboxylic acid or a physiologically acceptable salt thereof, for the manufacture of a medicament for the treatment of dyskinetic and choreatic syndromes.

7. Use of trans-4-[4-(3-chloro-4-methoxybenzylamino)-benzothieno-[2,3-d]pyrimidin-2-yl]cyclohexanecarboxylic acid or a physiologically acceptable salt thereof, for the manufacture of a medicament for the treatment of dystonic syndromes.

8. Use of trans-4-[4-(3-chloro-4-methoxybenzylamino)-benzothieno-[2,3-d]pyrimidin-2-yl]cyclohexanecarboxylic acid or a physiologically acceptable salt thereof, for the manufacture of a medicament for the treatment of extrapyramidal symptoms induced by neuroleptics

9. Use of trans-4-[4-(3-chloro-4-methoxybenzylamino)-benzothieno-[2,3-d]pyrimidin-2-yl]cyclohexanecarboxylic acid or a physiologically acceptable salt thereof, for the manufacture of a medicament for the treatment of tremor.

10. Use of trans-4-[4-(3-chloro-4-methoxybenzylamino)-benzothieno-[2,3-d]pyrimidin-2-yl]cyclohexanecarboxylic acid or a physiologically acceptable salt thereof, for the manufacture of a medicament for the treatment of extrapyramidal movement disorders chosen from the group consisting of Gilles de la Tourette syndrome, ballism, myoclonus, restless legs syndrome and Wilson's disease.

11. Pharmaceutical composition comprising, as active principles, (i) trans-4-[4-(3-chloro-4-methoxybenzylamino)-benzothieno-[2,3-d]pyrimidin-2-yl]cyclohexanecarboxylic acid or a physiologically acceptable salt thereof, and (ii) at least one anti-Parkinsonian drug, in combination with one or more pharmaceutically acceptable excipients.

12. Composition according to claim 11 for enhancing the anti-Parkinsonian effect of the anti-Parkinsonian drug.

13. Composition according to claim 11, in which (i) the active principle is in the form of its sodium or ethanolamine salt and (ii) the conventional anti-Parkinsonian drug is I-dopa.

14. Composition according to claim 11, in which (i) the active principle is in the form of its sodium or ethanolamine salt and (ii) the conventional anti-Parkinsonian drug is I-dopa combined with benserazide.

15. Composition according to claim 11, in which (i) the active principle is in the form of its sodium or ethanolamine salt and (ii) the conventional anti-Parkinsonian drug is I-dopa combined with carbidopa.

16. Use of trans-4-[4-(3-chloro-4-methoxybenzylamino)-benzothieno-[2,3-d)pyrimidin-2-yl]cyclohexanecarboxylic acid or a physiologically acceptable salt thereof in combination with at least one anti-Parkinsonian drug, for the preparation of a medicinal combination intended to enhance the anti-Parkinsonian effect of said anti-Parkinsonian drugs.
